(19) Europäisches Patentamt
European Patent Office
Office européen des brevets

(11) EP 1 739 422 A1

(12) DEMANDE DE BREVET EUROPEEN

(43) Date de publication:
**03.01.2007 Bulletin 2007/01**

(51) Int Cl.:
***G01N 33/18*** *(2006.01)*

(21) Numéro de dépôt: **06358010.4**

(22) Date de dépôt: **20.06.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **01.07.2005 FR 0507000**

(71) Demandeur: **Société des Eaux de Marseille**
**13006 Marseille (FR)**

(72) Inventeurs:
- **Boudenne, Jean-Luc**
  **13009 Marseille (FR)**
- **Coulomb, Bruno**
  **Av des Chabauds-RN8 13320 Bouc Bel Air (FR)**
- **Lieutaud, Gérard**
  **13008 Marseille (FR)**

(74) Mandataire: **Domange, Maxime**
**Cabinet Beau de Lomenie,**
**232, avenue du Prado**
**13295 Marseille Cedex 08 (FR)**

# (54) Procédé de traitement de déchloration des eaux en flux notamment en amont d'un détecteur biologique de pollution

(57) La présente invention concerne un procédé de traitement de déchloration sélective d'une eau en circulation contenant du chlore désinfectant sous forme $ClO^-$, HClO, $ClO_2$ ou chloramine, caractérisé en ce que l'on injecte une solution aqueuse d'acide ascorbique à partir d'un réservoir de stockage dans un dit flux d'eau à traiter, le débit massique d'acide ascorbique de ladite solution aqueuse d'acide ascorbique injectée (D1) étant réglé par rapport au débit massique de chlore, exprimé en $Cl_2$, dans l'eau à traiter en circulation (D2), de sorte que le rapport (R) desdits débits massique en acide ascorbique et chlore (R=D1/D2) soit compris entre 2,5 et 4, de préférence inférieur ou égal à 3.

La présente invention concerne plus particulièrement un procédé caractérisé en ce que l'on traite l'eau d'alimentation d'un détecteur biologique comprenant une teneur en chlore désinfectant incompatible avec la survie des espèces biologiques utilisées dans ledit détecteur et, plus particulièrement, ledit détecteur biologique est fondé sur le principe de détection des mouvements de poissons (par exemple des truitelles) dans une cuve et on maintient le taux de chlore désinfectant dans l'eau en circulation après mélange à une valeur inférieure ou égale à 0,6 mg/l, de préférence inférieure ou égale à la limite détectable.

EP 1 739 422 A1

## Description

La présente invention concerne un procédé de déchloration des eaux en flux, notamment en amont d'un détecteur biologique de pollution.

La présente invention concerne plus particulièrement le traitement d'eau contenant du chlore mis en oeuvre à titre d'agent désinfectant, à savoir au degré d'oxydation +1, sous forme $ClO^-$ et HClO et/ou sous forme de monochloramine $NHCl_2$, et/ou au degré d'oxydation +4 sous forme $ClO_2$.

La pollution des eaux, notamment de l'eau potable, peut être provoquée par une pluralité de substances plus ou moins toxiques et il n'est pas possible de détecter individuellement la présence de chacune de ces substances.

Pour surveiller la bonne qualité d'une eau potable, on met en oeuvre des détecteurs biologiques dont le principe consiste à s'assurer que des animaux très sensibles aux pollutions vivent normalement dans l'eau que l'on désire contrôler. Le choix des animaux se porte naturellement sur des poissons et le plus souvent sur les petites truites appelées "truitelles" qui sont très sensibles à la plupart des pollutions et dont la sensibilité est d'autant plus élevée qu'elles sont petites. La mort des petites truites constitue alors l'indice d'une pollution globale.

Le principe des détecteurs biologiques est donc fondé sur la détection de la survie d'espèces vivantes utilisées comme bio indicateur de la présence de polluant toxique.

Un dispositif connu consiste en un bac en forme de cuvette dans lequel vit une ou plusieurs truites et dans lequel on détecte la survie de la ou lesdites truites par différents moyens.

Dans FR 2 573 875, on a décrit un détecteur biologique permettant de détecter automatiquement la mort de tous les poissons vivants dans un bac, dans lequel l'eau à contrôler circule, de telle sorte que le signal émis par les moyens de détection puisse être utilisé pour déclencher une alarme et/ou un automatisme en éliminant les risques de fausse alarme.

Dans FR 2 573 875, on utilise plus précisément un détecteur volumétrique de mouvement visant à détecter l'absence de mouvement simultané de tous les poissons, détecteur volumétrique de mouvement composé d'un émetteur d'ondes acoustiques (sonar) et d'un ou plusieurs transducteurs électroacoustiques fonctionnant en récepteur et comportant en outre des circuits électroniques qui reçoivent les signaux électriques délivrés par ledit détecteur, lesquels circuits comportent un mélange qui reçoit les signaux délivrés par l'émetteur et par lesdits récepteurs, un filtre passe-bas dont la fréquence de coupure est inférieure à la fréquence dudit émetteur, un redresseur qui reçoit les signaux filtrés et qui délivre un signal continu à un signal de référence et qui actionne une alarme et/ou un automatisme si ledit signal continu devient inférieur audit signal de référence. Ce détecteur volumétrique de mouvement détecte en fait une absence simultanée de mouvement de tous les poissons et est suffisamment sensible aux mouvements des poissons pour éliminer les signaux parasites dus aux turbulences de l'eau. Ce dispositif dénommé "truitoSEM" est un outil de détection de traces polluantes et/ou toxiques dans les eaux brutes destinées à la production d'eau potable, basé sur l'observation optique du comportement de truitelles.

Les détecteurs biologiques de pollution existants sur le marché présentent l'inconvénient principal qu'ils ne sont pas adaptés à des eaux contenant des taux de composés chlorés utilisés comme agents désinfectants tels que mentionnés ci-dessus, incompatibles avec la survie des espèces vivantes utilisées comme bio indicateur de la présence de polluants toxiques, tels que poissons, bactéries, moules, daphnies.

En Europe, le chlore utilisé à titre d'agent désinfectant dans les eaux potables n'est autorisé que sous forme $ClO^-$, HClO, $Cl_2$ et $ClO_2$, les chloramines ($NHCl_2$) ne devant pas dépasser 0,1 mg/l. Au Canada et aux Etats-Unis, l'utilisation de monochloramine comme agent désinfectant est autorisée. Il convient d'observer ici que le chlore sous forme gazeuse $Cl_2$ peut être utilisé comme agent désinfectant, mais il n'est pas stable en solution et se transforme automatiquement en $ClO^{--}$ et HClO dans l'eau.

En particulier, il a été montré que les mouvements des indicateurs biologiques tels que des poissons, étaient altérés dès la présence de 0,7 mg/l exprimé en équivalent $Cl_2$, de chlore désinfectant sous forme $ClO^-$, $ClO_2$, $NHCl_2$, teneur qui aboutit au déclenchement d'un système d'alerte de type sonar, indépendamment de toute pollution effective. Des teneurs supérieures à 1,4 mg/l entraînent une létalité supérieure à 50%. Les teneurs en chlore dans les eaux potables font l'objet de réglementation dans les différents pays. Aux Etats-Unis, le taux de chlore admissible dans les eaux potables, s'élève à 4 mg/l, exprimé en équivalent $Cl_2$ pour le chlore sous forme $ClO_2$ et $ClO^-$/HClO, et à 2 mg/l pour les monochloramines, exprimé en $NHCl_2$ (soit 1,45 mg/l exprimé en équivalent $Cl_2$). Les doses de chlore entraînant les premiers effets gênants sur les truitelles sont donc bien inférieures aux doses rencontrées dans les eaux distribuées en Amérique du Nord.

Il existe différents produits chimiques connus permettant d'éliminer le chlore, tels que le sulfite de sodium ($Na_2SO_3$), bisulfite de sodium ($NaHSO_3$), le métabisulfite de sodium ($Na_2S_2O_5$), le dioxyde de souffre ($SO_2$), l'acide ascorbique ou l'ascorbate de sodium, le peroxyde d'hydrogène ($H_2O_2$), l'acide oxalique ($C_2O_4H_2$), le nitrite de sodium ($NaNO_2$), le thiosulfate de sodium ($Na_2S_2O_3$), le thiosulfate de calcium ($Ca_2S_2O_3$).

Le but de la présente invention est donc de fournir un procédé de contrôle et d'élimination sélective des composés chlorés utilisés comme agents désinfectants, à savoir des composés chlorés avec le chlore au degré d'oxydation +1, sous forme $ClO^-$, HClO ou monochloramine, et/ou avec le chlore au degré d'oxydation +4, sous forme $ClO_2$, présents dans les eaux en circulation alimentant les détecteurs biologiques de pollution ou aquariums, en des teneurs incompatibles avec la survie d'espèces vivantes.

Un but de la présente invention est, plus généralement, de fournir un procédé de traitement de déchloration sélective des eaux qui puisse être étendu à tout type d'eaux contenant du chlore désinfectant en des teneurs incompatibles avec la survie d'espèces vivantes et, plus particulièrement, du traitement des eaux destinées à la consommation humaine, en aval ou au sein d'usine de traitement d'eau potable.

Les inventeurs ont réalisés de nombreux essais pour tester les différents agents de déchloration connus mentionnés ci-dessus. Ces essais ont conduit à des échecs, soit parce que les produits ou les conditions de mise en oeuvre de ceux-ci se sont révélés toxiques pour les espèces vivantes et ne pouvaient donc être employés dans les systèmes biologiques de détection de pollution avec notamment modification du pH et/ou de la teneur en oxygène, soit encore parce que les réactifs chimiques se dégradaient au cours du temps ou encore parce que les réactifs n'agissaient pas assez rapidement.

En effet, un problème lié au traitement des eaux d'alimentation d'un détecteur biologique est que, dans le cas d'un traitement d'eau en circulation, le réactif doit permettre d'éliminer le chlore dans un système fonctionnant en flux et donc la cinétique de réaction avec le chlore doit être très rapide.

D'autre part, un autre but de la présente invention est de fournir un procédé de traitement qui nécessite une maintenance minimale avec en outre mise en oeuvre d'un produit soluble dans l'eau et injectable en continu.

Un autre problème est donc que le réactif ait une durée de vie ou efficacité suffisamment longue pour être compatible avec le renouvellement des espèces vivantes couramment utilisées dans les détecteurs biologiques de pollution, en général de 30 à 45 jours, afin d'assurer une maintenance minimale, de façon à ce que le système de traitement proposé permette de déchlorer des eaux en continu durant au minimum 30 jours, de préférence 45 jours, voire 60 jours, sans aucune intervention humaine.

Enfin et surtout, la mise en oeuvre du réactif ne doit pas masquer la présence de polluants toxiques déversés, volontairement ou non, dans les réseaux de distribution et donc maintenir la fonction et l'efficacité des détecteurs biologiques couramment utilisés comme station d'alerte pour ne pas conduire à l'élimination artificielle d'autres composés toxiques.

Le réducteur optimal pour la déchloration d'eau d'alimentation de détecteurs biologiques, par exemple à base de truitelles, doit donc remplir les conditions suivantes :

- stabilité d'au moins un mois,
- cinétique de réaction avec le chlore rapide,
- absence de toxicité pour les espèces vivantes, et notamment maintien des conditions physico chimiques de pH et de teneur en $O_2$ dissous,
- non génération de sous produits de réaction toxiques.

Le bisulfite de sodium semblait présenter la plupart des critères requis mais, dans les différentes conditions de mise en oeuvre testées, le bisulfite de sodium conduisait à une diminution brutale de l'oxygène dissous et du pH de l'eau lors de la réaction qui était sans doute la cause probable du décès des truitelles.

L'ascorbate de sodium semblait également un bon candidat, cependant, son manque de stabilité n'a pas permis de le retenir.

Les inventeurs ont dû réaliser de nombreuses études pour tester différents réactifs et conditions de stockage et de mise en oeuvre, et ont pu déterminer que, moyennant une régulation fine de l'ajout d'acide ascorbique en terme de concentration d'acide ascorbique et de débit d'injection dans le courant d'eau à traiter, les différents objectifs de la présente invention pouvaient être atteints.

La présente invention propose plus particulièrement un traitement de réduction desdits agents désinfectants chlorés, réduits sous forme $Cl^-$ à l'aide d'acide ascorbique par injection d'une solution d'acide ascorbique stockée dans un réservoir.

Selon la présente invention, on règle le rapport du débit massique d'acide ascorbique de la solution injectée par rapport au débit massique des dits composés chlorés désinfectants dans l'eau à traiter exprimés en équivalent $Cl_2$, de façon à le maintenir sensiblement stable à une valeur donnée comprise entre de 2,5 et 4.

Plus précisément, la présente invention fournit un procédé de traitement d'une eau en circulation contenant du chlore désinfectant sous forme $ClO^-$, HClO, $ClO_2$ et/ou $NHCl_2$, caractérisé en ce que l'on injecte une solution aqueuse d'acide ascorbique à partir d'un réservoir de stockage dans un dit flux d'eau à traiter, le débit massique d'acide ascorbique de ladite solution aqueuse d'acide ascorbique injectée D1 étant réglé par rapport au débit massique de dit chlore

désinfectant dans l'eau à traiter en circulation D2 exprimé en équivalent $Cl_2$, de sorte que le rapport R desdits débits massique en acide ascorbique et chlore désinfectant R=D1/D2 soit compris entre 2,5 et 4.

La présente invention permet un traitement de déchloration sélective au regard des seules espèces chlorées au degré d'oxydation +1 ou +4, à savoir sous forme $ClO_2$ (+4) ou $ClO^-$/HClO et/ou chloramine (+1), dans le cadre d'un traitement en flux d'une eau en circulation.

On entend donc ici par "chlore désinfectant", tout chlore libre ou combiné dans l'eau à traiter sous forme de $Cl_2$, $ClO^-$ , HClO, $ClO_2$ et monochloramine.

On peut ainsi réduire la teneur en chlore désinfectant à une valeur inférieure à 0,6 mg/l, voire inférieure à 0,1 mg/l exprimé en équivalent $Cl_2$, soit, en pratique, la limite de détection du chlore lors de l'analyse de l'eau en circulation.

Ces conditions de mise en oeuvre de la solution d'acide ascorbique et de régulation précise de l'ajout du réactif chimique permettent, comme il sera démontré plus loin, d'obtenir une réaction rapide avec un pouvoir réducteur suffisant. En effet, selon la présente invention, le rapport R supérieur à 2,5 correspond donc à des conditions de surdosage contrôlé par rapport au ratio stoechiométrique de réaction de l'acide ascorbique vis-à-vis desdites espèces chlorées, de façon à compenser, d'une part, la diminution de la cinétique de réaction résultant des conditions de mise en oeuvre en flux, ainsi que compenser, d'autre part, la dégradation de l'acide ascorbique en acide déhydroascorbique au cours du temps dans ledit réservoir de stockage, pendant une durée d'au moins 30 jours, voire jusqu'à 60 jours.

Les rapports massiques théoriques des réactions stoechiométriques, exprimés en équivalent $Cl_2$, sont de R=2,37 pour les monochloramines, et 2,48 pour les composés chlorés $ClO^-$, HClO et $ClO_2$.

Mais, ce surdosage est limité, à savoir inférieur à 4, voire de préférence inférieur ou égal à 3, pour s'assurer que la quasi-totalité de l'acide ascorbique soit bien consommé dans la réaction avec lesdites espèces chlorées et pour éviter une modification des paramètres physico-chimiques tels que pH ou concentration en $O_2$ dissous dans l'eau après réaction. Une telle réaction aurait un effet létal ou affecterait la vitalité des espèces vivantes et notamment des truitelles, et affecterait donc également le fonctionnement même des détecteurs biologiques ou aquariums en aval de l'eau en circulation à traiter.

Cette régulation précise de l'ajout du réactif chimique permet aussi de ne pas masquer la présence de polluants toxiques déversés volontairement ou non dans les réseaux de distribution et donc de maintenir l'efficacité des détecteurs biologiques couramment utilisés comme stations d'alerte. En effet, une régulation non précise de l'ajout d'acide ascorbique et notamment un ajout en excès aboutiraient non seulement à l'élimination du chlore et de ses dérivés mais également à l'élimination artificielle de composés toxiques. Il est donc nécessaire d'ajouter la quantité d'acide ascorbique en quantité exactement suffisante pour éliminer le chlore et ses dérivés, et uniquement le chlore et ses dérivés. Il faut donc maintenir une concentration en acide ascorbique juste nécessaire et suffisante pour réagir avec le chlore présent mais pas les autres produits toxiques. Ce surdosage limité permet notamment que l'acide ascorbique ne réagisse pas avec les espèces chlorées polluantes à détecter, telles que les chlorites ($ClO_3^-$) ou chlorates ($ClO_4^-$).

En outre, cette régulation précise permet, bien sûr, une économie de réactif.

Dans un mode de réalisation préférée, pour un traitement de déchloration sélective de l'eau contenant du chlore désinfectant, sensiblement exclusivement sous forme $ClO_2$, $ClO^-$, HClO et/ou monochloramine, le rapport R des débits massiques d'acide ascorbique D1 et de chlore D2, est maintenu de préférence sensiblement stable à une valeur donnée inférieure ou égale à 3, de préférence encore égale à 3.

De préférence, la concentration en acide ascorbique dans ladite solution aqueuse d'acide ascorbique injectée, est supérieure à 10% (100 g/l), de préférence encore supérieure ou égale à 20% (200 g/l). Cette caractéristique contribue à maintenir le pouvoir réducteur de l'acide ascorbique en évitant la transformation de l'acide ascorbique en acide déshydroascorbique notamment, pendant au moins 30 jours, voire jusqu'à 60 jours.

Selon d'autres caractéristiques avantageuses:

- ladite solution aqueuse d'acide ascorbique et ladite solution d'eau à traiter chlorée sont mélangées dans un mélangeur permettant l'homogénéisation rapide des deux fluides, de préférence un mélangeur statique, de préférence encore un mélangeur statique hélicoïdal,

- ledit réservoir de stockage de la solution aqueuse d'acide ascorbique est maintenu dans l'obscurité et à température ambiante dans une enceinte, de préférence une glacière à effet Peltier.

Les inventeurs ont découvert que les conditions d'utilisation et de stockage du réactif chimique et de ses dérivés, notamment les paramètres d'injection de ce composé (débit, proportions) et l'utilisation de différents moyens permettant la régulation de l'ensemble du procédé de déchloration sont déterminants pour remplir ces objectifs et résoudre les problèmes mentionnés ci-dessus.

Cette élimination en continu du chlore se doit en effet d'être régulée en fonction de la teneur en chlore, par la concentration d'acide ascorbique utilisée, par son débit d'injection et par le type de mélangeur utilisé (à adapter en fonction du débit de fonctionnement du détecteur biologique auquel cette invention est couplée).

Dans un mode de réalisation particulièrement avantageux, ladite solution aqueuse d'acide ascorbique est injectée à l'aide d'une pompe doseuse asservie à un analyseur de la concentration en chlore dans l'eau à traiter, de manière à régler le débit massique de ladite solution aqueuse d'acide ascorbique injectée en fonction de ladite concentration en chlore.

En pratique, on règle le débit volumique de ladite solution d'acide ascorbique injectée compte tenu de la concentration en acide ascorbique dans la solution aqueuse d'acide ascorbique stockée.

De préférence, ladite solution d'acide ascorbique est stockée pendant au plus 60 jours, de préférence au plus 30 jours.

Selon d'autres caractéristiques particulières :

- la concentration en dit chlore désinfectant de l'eau à traiter est de 0,1 à 5 mg/l, exprimée en équivalent $Cl_2$, et

- le débit volumique des eaux à traiter est de 100 à 1000 l/h.

Pour de tels débits volumiques et concentrations en chlore, le débit volumique en acide ascorbique est de l'ordre de 0,01 à 0,10 l/h.

Plus particulièrement encore, le pH de l'eau à traiter est de 6 à 8,5.

Ce procédé peut être étendu à tout type d'eau contenant du dit chlore désinfectant en des teneurs incompatibles avec la survie d'espèces vivantes.

L'invention permet d'élimination du dit chlore désinfectant présent dans des eaux traitées, destinées à la consommation humaine, en aval ou au sein d'usines de traitement d'eaux potables. Ce procédé permet d'éliminer ledit chlore désinfectant en flux en des temps très rapides et ceci quelles que soient les teneurs initiales en dit chlore désinfectant.

La présente invention est plus particulièrement avantageuse lorsque l'on traite l'eau d'alimentation d'un détecteur biologique, d'un aquarium ou d'un vivier, comprenant une teneur en chlore incompatible avec la survie des espèces biologiques utilisées dans ledit détecteur biologique, aquarium ou respectivement vivier.

Plus particulièrement encore, ledit détecteur biologique est fondé sur le principe de détection des mouvements de poissons (par exemple de truitelles) dans une cuve.

De préférence, on maintient le taux de dit chlore désinfectant dans l'eau en circulation après mélange à une valeur inférieure ou égale à 0,6 mg/l, de préférence encore, inférieure ou égale à la limite détectable de 0.1 mg/l exprimé en équivalent $Cl_2$.

L'invention permet d'étendre les applications de tout détecteur biologique utilisé à des fins de détection de pollutions accidentelles ou volontaires sur les réseaux de distribution d'eaux potables, et notamment à des eaux fortement chlorées, en composés chlorés incompatibles avec la survie des espèces biologiques couramment utilisées dans de tels systèmes.

Plus particulièrement, la présente invention concerne un procédé de détection de la pollution des eaux dans lequel on met en oeuvre le couplage d'un détecteur biologique et d'un traitement de déchloration des eaux en aval ou au sein des dits détecteurs.

D'autres caractéristiques et avantages de la présente invention apparaîtront à la lumière de la description qui va suivre, faite en référence aux figures annexées dans lesquelles :

- la figure 1 représente le pouvoir réducteur de l'acide ascorbique (%) en fonction du temps pour de l'acide ascorbique en concentration de 10% (100 g/l) et de l'acide ascorbique en concentration de 20% (200 g/l),

- la figure 2 représente les valeurs de pH (- - -) et teneur en oxygène dissous (—), ainsi que concentration en chlore dans l'eau traitée après réaction avec l'acide ascorbique en fonction du rapport R des débit massique en acide ascorbique de la solution injectée D1/ débit massique en chlore D2 dans l'eau à traiter, les concentrations en chlore (mg/l) ont été mesurées respectivement sur des solutions acides d'ascorbique stockées à t=0 (-o-o-) et t=60 jours (-□-□), et- la figure 3 représente un schéma d'installation d'un traitement selon l'invention en amont d'un détecteur biologique.

Le chlore est le désinfectant le plus utilisé pour l'élimination des germes pathogènes et pour la sécurité sanitaire du transport de l'eau dans les canalisations. Si l'on met du chlore dans l'eau, il pénètre à l'intérieur des bactéries en se diffusant au travers de la membrane cytoplasmique et y bloque l'activité enzymatique, ce qui a pour effet de détruire l'organisme en question. Les virus étant plus résistants, les conditions de traitement sont réglées en fonction de leur destruction. Le traitement est donc réglé par la dose et le temps de contact. Si on augmente la dose, il faut moins de temps de contact et inversement.

Le chlore peut se combiner dans l'eau de plusieurs façons. Ainsi, en fonction du pH de l'eau, s'établit un

équilibre entre 3 espèces chimiques:

- Le chlore à l'état moléculaire dissous, $Cl_2$,
- L'acide hypochloreux HClO,
- L'ion hypochlorite $ClO^-$

L'acide hypochloreux est 100 fois plus désinfectant que l'ion hypochlorite à un pH entre 4 et 6 parce qu'il peut traverser plus facilement la membrane cellulaire des micro-organismes et détruire les substances intracellulaires. Ce pH étant souvent plus élevé, il faut donc augmenter la dose et/ou le temps de contact.

Dès que l'on met du chlore dans l'eau, il réagit avec les composés azotés, dont notamment l'ammoniaque, avec lequel il se combine pour former des chloramines. Bien que plus stable, ces chloramines ont aussi un effet désinfectant, mais 5 fois moindre que l'ion hypochlorite et 500 fois moins que l'acide hypochloreux. Ce sont souvent ces chloramines qui donnent un goût désagréable à l'eau.

Les produits utilisés pour la désinfection au chlore sont: l'hypochlorite de sodium, l'hypochlorite de calcium, le dioxyde de chlore ou le chlore gazeux. Ce dernier est le plus utilisé, car plus facile d'exploitation. Il est stocké à l'état liquide dans des bouteilles ou réservoirs sous pression. Ceci permet d'obtenir 1,2 litre de chlore pour un litre de volume. Par comparaison, un litre d'hypochlorite de sodium à 48° (degré chlorométrique) ne contient que 150 g/l de chlore exprimé en équivalent $Cl_2$. Une bouteille de 50 kg de chlore équivaut à 400 kg d'hypochlorite de sodium. De plus, le chlore est stable à l'état liquide ou gazeux, à l'opposé des solutions d'hypochlorite de sodium. Ensuite, c'est sous forme gazeuse que le chlore est introduit dans l'eau, à l'aide d'un hydroéjecteur.

Un autre produit est de plus en plus utilisé ; il s'agit du dioxyde de chlore $ClO_2$. Celui-ci à l'avantage d'avoir une efficacité bien supérieure au chlore gazeux et aux hypochlorites. Il est 10 fois plus soluble dans l'eau et a un effet rémanent plus marqué. Il permet d'éliminer efficacement les substances humiques et même les phénols, les oxydes le fer et de manganèse plus rapidement, et de lutter contre les algues et cellules pathogènes, tout cela dans une large gamme de pH. L'inconvénient réside essentiellement dans les installations nécessaires à la production et la chloration.

En Amérique du Nord, dans le passé, la monochloramine inorganique ($NH_2Cl$) a été largement utilisée, mais elle ne l'est plus que rarement aujourd'hui à cause d'une cinétique d'oxydation lente par rapport aux substances mentionnées ci-dessus, et à cause de l'incertitude quant au risque qu'elle pose pour la santé humaine.

Dans le domaine de pH des eaux potables, ce sont donc les formes HClO et $ClO^-$ (appelées "chlore libre") qui sont présentes. La répartition de ces deux formes va dépendre du pH, de la température et de la présence d'espèces oxydables.

Les teneurs en chlore désinfectant dans les réseaux de distribution nord américains (3 à 4 mg/l) exprimé en équivalent $Cl_2$ sont beaucoup plus élevés qu'en Europe (0,1 à 0,2 mg/l); ces teneurs sont incompatibles avec la survie de poissons, comme cela a déjà été démontré dans de nombreuses publications (la teneur en chlore résiduel total doit être inférieure à 0,015 mg/l, exprimé en HClO.

Plusieurs études portant sur des poissons ont montré que le chlore libre ou combiné peut avoir des effets nocifs sur la structure des branchies et sur la capacité de l'hémoglobine de transporter l'oxygène dans le sang. Les dommages subis par les membranes branchiales et l'oxydation de l'hémoglobine en méthémoglobine portent à croire que la cause finale de la mortalité découlant de l'exposition au chlore libre ou combiné est l'asphyxie. En outre, selon plusieurs études, le chlore libre et le chlore combiné pourraient exercer leur toxicité sur d'autres sites, dont le système nerveux a montré que l'exposition au chlore résiduel augmentait la perméabilité des branchies, ce qui pouvait mener à une augmentation de l'accumulation (et, par là même, de la toxicité) d'autres substances chimiques présentes dans les eaux usées chlorées.

Cette toxicité du chlore pour les truitelles a été également démontrée par les inventeurs. Douze truitelles ont été maintenues dans un aquarium contenant de l'eau chlorée par des doses croissantes d'hypochlorite de sodium. Chaque dose de chlore a été maintenue pendant une durée de 8 heures. L'aquarium a été placé sous aération permanente à l'aide d'une pompe à air munie d'un diffuseur poreux. Le tableau suivant présente les résultats observés lors de cette expérimentation.

Le tableau suivant présente les résultats observés lors de cette expérimentation. ($[Cl_2]$= chlore désinfectant exprimé en équivalent $Cl_2$)

| $[Cl_2]$ (en mg/l) | Effets observés | % mortalité observée |
|---|---|---|
| 0,25 | aucun effet | 0 |
| 0,70 | diminution de la mobilité ventilation accélérée | 10 |

(suite)

| $[Cl_2]$ (en mg/l) | Effets observés | % mortalité observée |
|---|---|---|
| 1,4 | mobilité très réduite perte d'appétit ventilation très importante diminution de la taille de l'iris | 60 |
| 2,1 | nage sur le côté puis sur le dos mort | 100 |

Il apparaît que les doses de chlore désinfectant supérieures à 2 mg/l exprimé en équivalent $Cl_2$ sont létales pour les truitelles. D'autre part, la mobilité des truitelles semble affectée très rapidement, par des doses en chlore supérieures ou égales à 0,7 mg/l. Cette perte de mobilité pourrait entraîner un déclenchement d'alarme par le capteur acoustique du type sonar d'un détecteur biologique TruitoSEM (non utilisé dans cette étude).

Les doses de chlore entraînant les premiers effets gênants sur les truitelles sont donc bien inférieures aux doses rencontrées dans les eaux distribuées en Amérique du Nord. Il apparaît donc important de procéder à une déchloration de l'eau en cas d'installation d'un détecteur biologique TruitoSEM sur ces sites.

Les premiers essais au bisulfite de sodium s'étant révélés infructueux, différents essais ont été réalisés afin de proposer un agent réducteur plus adapté à l'utilisation d'un détecteur biologique. En effet, le produit à retenir doit :

- ne pas réagir ou très peu réagir avec l'oxygène dissous du milieu ;
- ne pas influencer fortement le pH ;
- ne pas être toxique pour les truitelles ;
- ne pas générer des sous-produits toxiques.

Une fois l'agent réducteur choisi, des expérimentations ont été menées, tout d'abord en mode statique ("batch") pour déterminer les conditions opératoires optimales, puis en conditions réelles (en « flux ») en présence de truitelles, à l'aide d'un détecteur biologique.

Le schéma réactionnel de l'acide ascorbique est complexe et peut conduire à différentes espèces chimiques suivant les conditions aérobies ou anaérobies du milieu. Les conditions aérobies conduisent essentiellement à la production de réductones selon le schéma suivant:

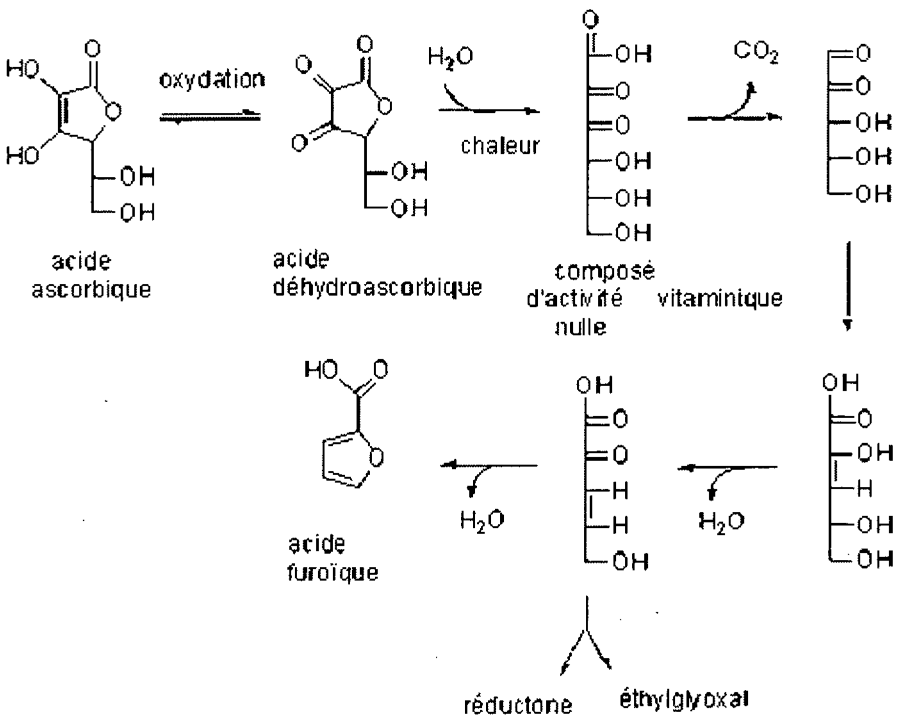

Cette oxydation de l'acide ascorbique est favorisée par la chaleur, la lumière, un milieu acide (conditions optimales : T=100°C et milieu $H_2SO_4$ à 5%) ou la présence de souches bactériennes réalisant l'acide ascorbique oxydase.

Le suivi d'une solution d'acide ascorbique par spectrophotométrie d'absorption moléculaire dans le domaine ultraviolet montre ainsi une modification de spectre et donc de forme chimique au cours du temps, probablement par l'effet combiné de la chaleur et de la lumière. On peut observer sur les spectres un décalage de longueur d'onde entre l'acide ascorbique et un sous-produit de dégradation.

Cependant, les espèces chimiques issues de la dégradation de l'acide ascorbique peuvent garder un fort pouvoir réducteur.

D'après les réactions de réduction ci-dessous de l'acide hypochloreux ou de l'ion hypochlorite par l'acide ascorbique, oxydant et réducteur réagissent mole à mole.

$$C_5H_5O_5CH_2OH + HClO \rightarrow C_5H_3O_5CH_2OH + HCl + H_2O$$

$$C_5H_5O_5CH_2OH + NaClO \rightarrow C_5H_3O_5CH_2OH + NaCl + H_2O$$

A pH=8, le chlore dans l'eau est présent à 80% sous la forme hypochlorite ($ClO^-$) et à 20% sous la forme acide hypochloreux (HClO).

La quantité d'acide ascorbique nécessaire à la réduction d'une concentration donnée d'hypochlorite de sodium a été vérifiée par expérimentations. Les essais ont été réalisés sur des solutions d'acide ascorbique et d'eau de javel ($Na^+$, $ClO^-$) équimolaires et égales à $1{,}55.10^{-4}$ mole/litre, soit respectivement 27,34 ppm et 8 ppm, pour faciliter le suivi des réactions. Un taux d'efficacité de 100% est atteint dès la mise en oeuvre d'une réaction mole à mole (rapport 1).

Ce premier résultat permet de calculer la dose théorique d'acide ascorbique à injecter pour déchlorer l'eau. En partant d'une dose de chlore résiduelle maximale (exprimée en $Cl_2$) de 4 mg/l dans les eaux distribuées en Amérique du Nord (soit 2,9 mg/l ou $5{,}63.10^{-5}$ $mol.L^{-1}$ de $ClO^-$), la déchloration sera effective avec $5{,}63.10^{-5}$ $mol.L^{-1}$ d'acide ascorbique, soit 9,92 mg/l. Cette réaction produira par ailleurs 2 mg/l d'ions chlorures. On a donc un rapport 2,48 entre la quantité de chlore présente et la quantité d'acide ascorbique nécessaire.

D'après la notice d'utilisation du détecteur biologique TruitoSEM® de la société Cifec, le débit d'eau dans le système doit être de 700 l/h, réductible à 200 l/h. Si on considère toujours une eau chlorée à 4 mg/l de $Cl_2$, on aura donc un flux de chlore compris entre 800 et 2800 ppm de $Cl_2$ par heure respectivement pour les débits de 200 et 700 l/h. Il faudra donc un flux de 1984 à 6944 ppm d'acide ascorbique par heure. Si on utilise une solution d'acide ascorbique à 10% (soit 100 g/l), il faudra en injecter 19,8 à 69,5 ml par heure (soit environ 6,6 à 23,2 ml par heure pour une solution d'acide ascorbique à 30%, proche de la solubilité maximale de l'acide ascorbique trouvée à 333 g/l dans la littérature). Cependant, les faibles débits d'acide ascorbique nécessaires posent un problème de pompe doseuse. Il s'agira donc d'intégrer au système une nouvelle pompe doseuse pouvant desservir en continu les faibles débits nécessaires.

La stabilité de l'acide ascorbique, et plus précisément le maintien de son pouvoir réducteur, est un paramètre important dans le cadre d'une adaptation sur TruitoSEM®. En effet, le système doit pouvoir fonctionner sans intervention ni maintenance régulière de la part des utilisateurs. Comme nous l'avons vu précédemment, le suivi spectrophotométrique d'une solution d'acide ascorbique montre une modification de la forme chimique au cours du temps, probablement par l'effet combiné de la chaleur et de la lumière. Cependant, le schéma réactionnel de l'acide ascorbique est complexe et peut conduire à des espèces chimiques conservant un fort pouvoir réducteur. Ce pouvoir réducteur a été vérifié au cours du temps par iodométrie (dosage volumétrique), pour d'une solution d'acide ascorbique à 10%, conservée à l'obscurité et à température ambiante du laboratoire (environ 20°C).

Les essais montrent une faible diminution du pouvoir réducteur au cours du temps (-10% en 35 jours). Une solution d'acide ascorbique à 10% peut donc être utilisée sur une période de 35 jours si elle est à l'abri de la lumière. Cette période est augmentée dans le cas d'une utilisation d'une solution d'acide ascorbique plus concentrée (20%).

Afin d'assurer une déchloration efficace, il est intéressant d'utiliser un pilotage du système par un analyseur de chlore (pompe doseuse asservie à un analyseur de chlore). Toutefois, pour éviter ce pilotage par pompe doseuse, il est possible de travailler avec un léger excès d'acide ascorbique. L'acide ascorbique n'est pas directement toxique pour les truitelles, cependant la réaction d'oxydoréduction de déchloration peut entraîner une diminution du pH et/ou de l'oxygène dissous. Des expérimentations ont donc été réalisées en ce sens sur une eau distribuée, chlorée artificiellement, pour vérifier ces effets.

D'après les essais ci-dessus, un excès d'acide ascorbique a un effet relativement limité sur le pH de l'eau traitée. Dans des conditions avoisinant celles pouvant être rencontrées sur les eaux distribuées en Amérique du Nord, on a constaté une diminution maximale de 10% du pH pour un dosage d'acide ascorbique double par rapport à la stoechiométrie. Il est à noter que le pH reste tout de même dans une gamme favorable à la survie des truitelles, et ce quel que soit le pH initial de l'eau distribuée. De même, dans ces conditions, l'excès d'acide ascorbique a entraîné une baisse maximale d'environ 10% de l'oxygène dissous.

L'acide ascorbique semble donc être efficace pour la déchloration des eaux traitées. En effet, toutes les expérimentations menées à la stoechiométrie ou en léger excès de réducteur ont permis d'obtenir des doses en chlore résiduel inférieures à la limite de détection (< 0,1 mg/l).

D'autre part, l'acide ascorbique peut être utilisé en excès, et en se basant sur le domaine de pH optimal pour les truitelles (6 à 8), le traitement à l'acide ascorbique pourrait être utilisé pour des eaux potables présentant un pH compris entre 6 et 8,5, couvrant une grande partie de la gamme de pH autorisé pour les eaux distribuées (6 < pH < 9).

L'acide L-ascorbique (ou "vitamine C") est un produit aux propriétés réductrices qui réagit avec le chlore selon la réaction suivante :

$$C_5H_5O_5CH_2OH + (H^+,ClO^-) \rightarrow C_5H_3O_5CH_2OH + (H^+,Cl^-) + H_2O$$

Ce produit chimique se dégrade au cours du temps si des précautions ne sont pas prises. Il faut donc l'utiliser dans des conditions particulières qui ont été déterminées au cours des essais réalisés par les inventeurs:

- l'acide ascorbique doit être conditionné à une concentration proche de sa solubilité maximale (333 g/l) ;
- il doit être maintenu à l'obscurité et à une température inférieure à 25°C (l'acide ascorbique se dégrade à la lumière et à température élevée).

Dans ces conditions, il a été montré que l'acide ascorbique se dégradait au cours du temps en acide déhydroascorbique, mais ce sous-produit présente une activité réductrice équivalente à celle de l'acide ascorbique initial.

Ainsi, dans les conditions établies par les essais réalisés, le pouvoir d'action de l'acide ascorbique (ou de ses sous-produits) envers le chlore est total durant 60 jours.

Si ces conditions ne sont pas respectées, d'autres sous-produits sont générés (acide furoïque, éthylglyoxal, réductones, ...), composés présentant une activité réductrice moindre.

La concentration du produit et les débits d'injection sont apparus déterminants.

Le taux d'acide ascorbique doit être régulé par la mesure de la teneur en chlore dans les eaux à traiter (mesure de chlore en ligne à l'aide d'appareils commerciaux) ou fixé si l'on connaît la valeur moyenne en chlore présente dans les eaux à traiter.

Les teneurs en acide ascorbique à ajouter pour éliminer tout le chlore dans les eaux à traiter sont données par la relation:

9,92 mg/l d'acide ascorbique pour 4 mg/l de chlore sous forme $ClO^-$, HClO ou $ClO_2$ (exprimé en $Cl_2$).

Le rapport optimal théorique stoechiométrique de concentration acide ascorbique/$Cl_2$ doit être de 2,48.

Les débits d'injection de l'acide ascorbique pour éliminer le chlore vont dépendre des débits de fonctionnement des détecteurs biologiques de pollution pour lesquels ce procédé est destiné, et devront être régulés par l'emploi d'une pompe doseuse.

Il est apparu que le type de mélangeur qui est utilisé en amont du détecteur biologique, est important, ce mélangeur doit permettre un mélange parfait de l'acide ascorbique avec l'eau à traiter.

Le produit utilisé pour éliminer le chlore dans l'eau peut être utilisé sur une période plus longue (>60 jours); mais, dans ce cas, une mesure de sa présence peut s'avérer nécessaire. Un module de détection par sonde redox ou par spectrophotométrie peut donc être ajouté pour mesurer la quantité de réactif encore présente après ce délai optimal de fonctionnement.

Le procédé développé est optimal pour traiter des eaux dont le pH est compris entre 6 et 8,5. Une mesure de pH en entrée du dispositif peut donc permettre de valider le bon fonctionnement du procédé proposé.

Ces deux dispositifs ne sont en aucun cas obligatoires pour le bon fonctionnement du procédé développé mais peuvent permettre d'élargir la gamme des eaux à traiter et adapter les quantités de réactifs utilisés.

De même, la solution d'acide ascorbique doit être maintenue à l'obscurité à une température inférieure à 25°C susceptible d'être parfois dépassée selon le local d'utilisation. La fabrication sur mesure, en fonction des dimensions du réservoir de solution, d'une glacière à effet Peltier est avantageuse. Cette glacière permet d'atteindre 25 °C en dessous de la température ambiante, donc d'utiliser cet appareillage jusqu'à une température ambiante de 25°C + 25°C = 50°Celsius, température évidemment maximale à l'ombre de la quasi totalité des lieux possible d'utilisation d'un tel appareillage. Il est à noter que, l'effet Peltier étant réversible, cette même glacière permet éventuellement de réchauffer au dessus du point de gel la réserve de solution d'acide ascorbique en période de grand froid.

Le procédé développé a été validé sur plusieurs points :

1) Cinétique de réaction entre l'acide ascorbique et le chlore à éliminer.

Le procédé doit permettre d'éliminer le chlore dans un système fonctionnant en flux; la cinétique de réaction doit donc être très rapide. Pour que la réaction se fasse dans les meilleures conditions, il faut que le mélangeur, utilisé pour mettre en contact le chlore et l'acide ascorbique, permette l'homogénéisation rapide des deux fluides.

Les inventeurs ont utilisé un mélangeur statique en ligne comprenant des éléments de forme hélicoïdale, en polyacétal (de la société BIOBLOCK). Grâce à la géométrie hélicoïdale de ses éléments, le flux entrant dans le mélangeur est séparé en 2, un vortex est crée au sein de l'élément hélicoïdal, puis le flux est à nouveau séparé en deux par l'élément hélicoïdal suivant mais en sens inverse. Cette alternance de mouvement garantit un mélange homogène (12 éléments hélicoïdaux insérés en série dans un tuyau de diamètre interne 1,3 cm). Il a été prouvé qu'un tel système permettait l'élimination totale du chlore dès mélange avec l'acide ascorbique.

2) Efficacité de l'acide ascorbique avec le temps.

Comme montré sur la figure 1, si l'acide ascorbique est conservé dans des conditions optimales, son efficacité pour éliminer le chlore reste optimale durant 60 jours si la concentration en acide ascorbique est au moins de 20% (200 g/l). Il a été prouvé que l'action d'élimination du chlore était en fait liée à la fois à l'acide ascorbique et à son sous-produit, l'acide déhydroascorbique. Ainsi, après 60 jours d'utilisation dans les conditions d'injection définies, l'efficacité du produit reste d'environ 95%.

3) Influence de la réaction sur les paramètres physico-chimiques (pH et $O_2$ dissous) et optimisation du débit d'injection d'acide ascorbique.

Etant donné les faibles débits d'acide ascorbique à injecter dans le système, une pompe doseuse précise pour les faibles débits a été installée (pompe Stepdos KNF). Cette pompe permet d'injecter une solution d'acide ascorbique à des débits compris entre 0,03 et 30 ml par minute. Cependant, pour que la déchloration soit optimale, il est nécessaire que le mélange d'eau chlorée, arrivant avec un fort débit, et d'acide ascorbique, arrivant en continu selon un faible débit, soit maximal. Pour cela, la solution mise en oeuvre sur le montage expérimental est un mélangeur en ligne mentionné ci-dessus constitué d'un tuyau en PVC renforcé garni de plusieurs éléments hélicoïdaux en polyacétal. Le nombre d'éléments (Re) à insérer dans le tuyau a été déterminé par l'équation de Reynolds:

$$\mathrm{Re} = \frac{4705 D * \Delta}{\mu * \theta}$$

avec D le débit en litres/minute, $\Delta$la densité du fluide, $\mu$ sa viscosité, et $\theta$ le diamètre interne du tuyau en cm.

L'application de cette formule à cet exemple (tuyau de 1,3 cm de diamètre interne) donne un nombre de Reynolds Re > 1000 et donc un nombre d'éléments requis de 6 à 12.

L'eau déchlorée à la sortie du mélangeur alimente directement l'aquarium, équipé de deux sondes pour mesurer en temps réel l'oxygène dissous, le pH et la température. L'aquarium est maintenu sous aération permanente afin de ne pas induire de stress des poissons dû au manque d'oxygène dissous.

Dans un premier temps, un test en absence de truitelle a permis de régler le débit de la pompe doseuse pour l'injection d'acide ascorbique. Le graphique de la figure 2 montre que pour une dose initiale en chlore de 4,5 mg/l exprimé en équivalent $Cl_2$, un rendement de déchloration proche de 100% est obtenu pour un débit d'acide ascorbique à 20% de 0,030 l/h. Ce débit est supérieur à la stoechiométrie de la réaction acide ascorbique/$Cl_2$ et correspond à un rapport R=D1/D2 de 2,5 sur la figure 2.

Les courbes pH et $O_2$ se coupent d'ailleurs à ce point. A partir de ce débit, le pH commence à diminuer, l'oxygène restant stable.

Ces deux paramètres ($O_2$, pH) sont essentiels à surveiller pour la survie des poissons : toute modification trop importante de ces paramètres entraînerait des effets négatifs sur le mouvement des poissons, voire des effets létaux si ces paramètres chutent brutalement.

Au point de vue survie des poissons uniquement, cela veut dire qu'il ne faut pas aller au-delà d'un débit de 0,048 l/h d'acide ascorbique R= 4 (sinon variation de pH>0,2 unités). Au point de vue fonctionnement d'un détecteur biologique, il ne faut pas que l'acide ascorbique soit trop en excès car sinon il réagirait avec des produits toxiques (il masquerait la présence de ces composés), et donc il faut être le plus près possible du débit minimum d'acide ascorbique nécessaire et juste nécessaire pour réagir avec le chlore présent.

D'autre part, on n'observe aucun effet de l'acide ascorbique sur la teneur en oxygène dissous, et un effet modéré sur le pH jusqu'à un débit de 0,060 l/h (R= D1/D2=5 sur la figure 2), soit le double de la dose d'acide ascorbique nécessaire.

La même expérimentation a été reproduite avec une solution d'acide ascorbique stockée pendant 60 jours à l'abri de la lumière et à température ambiante. On peut constater que la déchloration est efficace pour un débit d'acide ascorbique légèrement supérieur au réglage optimal précédemment déterminé (0,036 l/h (R=3) au lieu de 0,030 l/h (R=2,5)). L'acide ascorbique garde donc un fort pouvoir réducteur s'il est stocké dans de bonnes conditions.

Ces essais ont été menés avec le même acide ascorbique qui avait été utilisé lors de l'expérience précédente (conservé dans les conditions de température et d'obscurité).

On constate qu'il faut travailler à un plus fort débit que précédemment pour éliminer le chlore initialement présent. Néanmoins, la variation de débit est faible : on passe de 0.03 l/h à 0.036 l/h, soit R=2,5 à R=3; cette variation de débit s'explique par la légère perte d'activité réductrice de l'acide ascorbique au bout de 60 jours (activité réduite de 5%; cf. courbe figure 1, mais comme on peut le constater, débit augmenté de 16%). Cet écart (5%-16%) s'explique par une diminution de la cinétique de réaction:l'activité réductrice est réduite (de 5%) et la vitesse avec laquelle cet acide ascorbique ("vieux" de 60 jours) réagit avec le chlore est diminuée de 16 %.

Tout cela justifie le fait que si le système de déchloration est destiné à des détecteurs biologiques pour lesquels la réserve d'acide ascorbique est changée tous les 60 jours, il vaut mieux opérer à un débit de sûreté de 0,036 l/h (R=3) (et en tout état de cause entre 0,03 et 0,036 l/h, soit un rapport de débit massique [acide ascorbique]/ [$Cl_2$] R de 2,5 à 3). Au-delà de 3, on risque de masquer la présence d'autres composés toxiques.

Pour déchlorer une eau contenant environ 4 mg/l de chlore désinfectant exprimé en équivalent $Cl_2$, il est donc possible d'utiliser une solution d'acide ascorbique à 20% pendant plusieurs semaines. Un débit de 0,048 l/h (rapport R=4 sur la figure 2) d'acide ascorbique permettrait de travailler en léger excès et ainsi d'anticiper la dégradation du réducteur, ainsi qu'une éventuelle augmentation brutale de charge en chlore dans l'eau traitée. Ce débit n'a en outre que très peu d'incidence sur les conditions physico-chimiques de l'eau dans l'aquarium (pH et $O_2$ dissous). Cependant cet excès d'acide ascorbique entraînerait une surconsommation de 30% de réducteur, et donc des coûts non négligeables. Dans les conditions opératoires utilisées lors de cette étude, la quantité d'acide ascorbique nécessaire à la déchloration serait d'environ 23 litres par mois pour un débit de 0,030 l/h et de 35 litres pour un débit de 0,048 l/h (R=4).

Ces tests de déchloration en flux ont dans un deuxième temps été menés en présence de truitelles, pendant une période continue de plusieurs jours. Les truitelles n'ont montré aucun signe physique anormal et une mobilité normale.

Comme mentionné ci-dessus, il est intéressant de travailler en léger excès d'acide ascorbique. Cependant, il est nécessaire d'évaluer si l'acide ascorbique peut induire une perturbation du comportement des truitelles. Parallèlement aux essais de déchloration en flux, un aquarium témoin a donc été mis en place, avec 6 truitelles vivant en permanence dans une eau ne contenant que de l'acide ascorbique en large excès (75 mg/l). Comme pour le TruitoSEM, l'aquarium a été maintenu sous aération constante. Les observations sur 15 jours n'ont montré aucun changement significatif dans le comportement des truitelles.

L'évolution comparée des teneurs en $O_2$, $Cl_2$ et pH représentée sur la figure 2 montre que le rapport optimal des débits massiques en acide ascorbique/chlore (R=D1/D2) doit être de 2,5 à 4 ce qui correspond à un débit optimal d'injection d'acide ascorbique de 0,03 à 0,048 l/h dans les conditions utilisées dans cet exemple d'application , à savoir : concentration d'acide ascorbique à 200 g/l ; débit maximal de fonctionnement du détecteur biologique 700 l/h ; débit d'entrée dans le détecteur après mélangeur de 160 l/h, et taux de chlore dans l'eau à traiter de 4,5 mg/l.

Un rapport R=D1/D2 de 3 correspondant à un débit de 0,036 l/h dans les conditions ci-dessus est optimal car il permet de rester proche du rapport stoechiométrique théorique tout en présentant un pouvoir réducteur quasi total pour une solution d'acide stockée 60 jours.

Cette fourchette permet d'obtenir un pouvoir réducteur quasi-total, les concentrations en $Cl_2$ mesuré étant à la limite de détection tout en préservant le pH et la teneur en $O_2$ qui sont les éléments déterminants pour juger de la toxicité et de la réaction éventuelle avec d'autres polluants.

4) Validation d'une installation de traitement selon le procédé couplé à un détecteur biologique.

Le détecteur biologique de pollution décrit dans FR 2 573 875, dénommé "TruitoSEM" avec une cuve comprenant une douzaine de truitelles, type Fario, a été utilisé pour valider le procédé développé avec une installation selon la figure 3 dans laquelle le réservoir de stockage 1 de la solution d'acide ascorbique est conservé à l'obscurité dans une enceinte du type glacière à effet Peltier avant d'être injectée à un débit massique D1 (mg/h) dans ladite solution d'eau chlorée à traiter circulant à un débit massique D2 (mg/h) pour y être mélangée dans un mélangeur statique hélicoïdal 2 avant d'alimenter la cuve de 3 du détecteur.

Ainsi le procédé présenté ci-dessus a permis d'éliminer le chlore malgré une teneur à l'entrée de 4,5 mg/l de $Cl_2$ dans l'eau à traiter. Pour un débit volumique d'eau entrant dans le détecteur de 160 l/h, un débit volumique d'acide ascorbique à 20% de 0,036 l/h a permis cette neutralisation complète pour une solution d'acide stockée entre 0 et 60 jours.

Cette expérimentation de longue durée (8 h/jour pendant vingt jours) en conditions réelles de fonctionnement du détecteur biologique n'a engendré aucune mortalité et n'a montré aucune toxicité sur les indicateurs biologiques présents.

Par contre un essai final de suppression de l'injection d'acide ascorbique a entraîné la mort immédiate des truitelles du fait d'une teneur en chlore de l'eau à 4,5 mg/l.

**Revendications**

1. Procédé de traitement de déchloration d'une eau en circulation contenant du chlore désinfectant sous forme $ClO^-$, HClO, $ClO_2$ et/ou monochloramine, **caractérisé en ce que** l'on injecte une solution aqueuse d'acide ascorbique à partir d'un réservoir de stockage (1) dans un dit flux d'eau à traiter, le débit massique d'acide ascorbique de ladite solution aqueuse d'acide ascorbique injectée (D1) étant réglé par rapport au débit massique de dit chlore désinfectant dans l'eau à traiter en circulation (D2) exprimé en équivalent $Cl_2$, de sorte que le rapport (R) desdits débits massique en acide ascorbique et chlore (R=D1/D2) soit compris entre 2,5 et 4.

2. Procédé selon la revendication 1, **caractérisé en ce que** la concentration en acide ascorbique dans ladite solution aqueuse d'acide ascorbique injectée, est supérieure ou égale à 20%.

3. Procédé selon l'une des revendications 1 ou 2, **caractérisé en ce que** ladite solution d'acide ascorbique est stockée pendant au moins 30 jours, de préférence jusqu'à 60 jours.

4. Procédé selon l'une des revendications 1 à 3, **caractérisé en ce que** ledit rapport (R) des débits massiques d'acide ascorbique (D1) et de chlore (D2), est maintenu sensiblement stable à une valeur donnée inférieure ou égale à 3 , de préférence encore égale à 3, et l'on réalise un traitement de déchloration sélective d'une eau contenant des dits composés chlorés désinfectants sous forme $ClO_2$, $ClO^-$, HClO et/ou monochloramine ($NH_2Cl$).

5. Procédé selon l'une des revendications 1 à 4, **caractérisé en ce que** ladite solution aqueuse d'acide ascorbique et ladite solution d'eau à traiter chlorée sont mélangées dans un mélangeur (2) permettant l'homogénéisation rapide des deux fluides, de préférence un mélangeur statique, de préférence encore un mélangeur statique hélicoïdal.

6. Procédé selon l'une des revendications 1 à 5, **caractérisé en ce que** ledit réservoir de stockage (1) de la solution aqueuse d'acide ascorbique est maintenu dans l'obscurité et à température ambiante dans une enceinte, de préférence une glacière à effet Peltier.

7. Procédé selon l'une des revendications 1 à 6, **caractérisé en ce que** ladite solution aqueuse d'acide ascorbique est injectée à l'aide d'une pompe doseuse asservie à un analyseur de la concentration en chlore dans l'eau à traiter, de manière à régler le débit massique de ladite solution aqueuse d'acide ascorbique injectée en fonction de ladite concentration en chlore.

8. Procédé selon l'une des revendications 1 à 7, **caractérisé en ce que** la concentration en dit chlore désinfectant de l'eau à traiter est de 0,1 à 5 mg/l, exprimée en équivalent $Cl_2$.

9. Procédé selon l'une des revendications 1 à 8, **caractérisé en ce que** le débit volumique des eaux à traiter est de 100 à 1000 l/h.

10. Procédé selon l'une des revendications 1 à 9, **caractérisé en ce que** le pH de l'eau à traiter est de 6 à 8,5.

11. Procédé selon l'une des revendications 1 à 10, **caractérisé en ce que** l'on traite l'eau d'alimentation d'un détecteur biologique de pollution (3), d'un aquarium ou d'un vivier, comprenant une teneur en chlore incompatible avec la survie des espèces biologiques utilisées dans ledit détecteur, aquarium ou respectivement vivier.

12. Procédé selon la revendication 11, **caractérisé en ce que** ledit détecteur biologique est fondé sur le principe de détection des mouvements de poissons dans une cuve.

13. Procédé selon la revendication 12, **caractérisé en ce que** l'on maintient le taux de dit chlore désinfectant dans l'eau en circulation après mélange à une valeur inférieure ou égale à 0,6 mg/l, de préférence inférieure ou égale à la limite détectable de 0,1 mg/l exprimé en équivalent $Cl_2$.

FIG.1

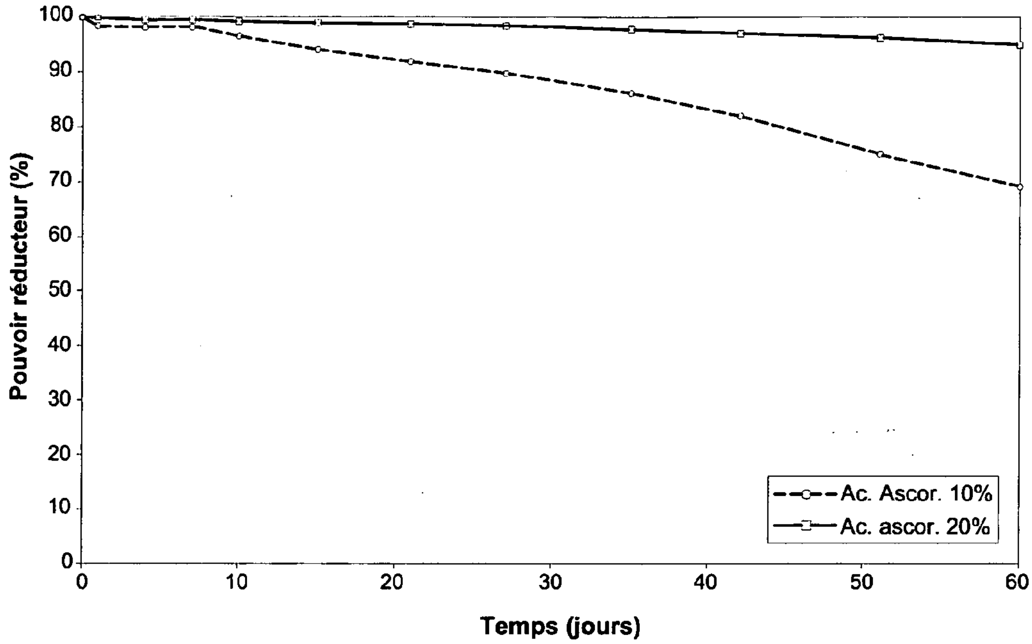

100
90
80
70
60
50
40
30
20
10
0
Pouvoir réducteur (%)
0
10
20
30
40
50
60
Temps (jours)
Ac. Ascor. 10%
Ac. ascor. 20%


FIG.2

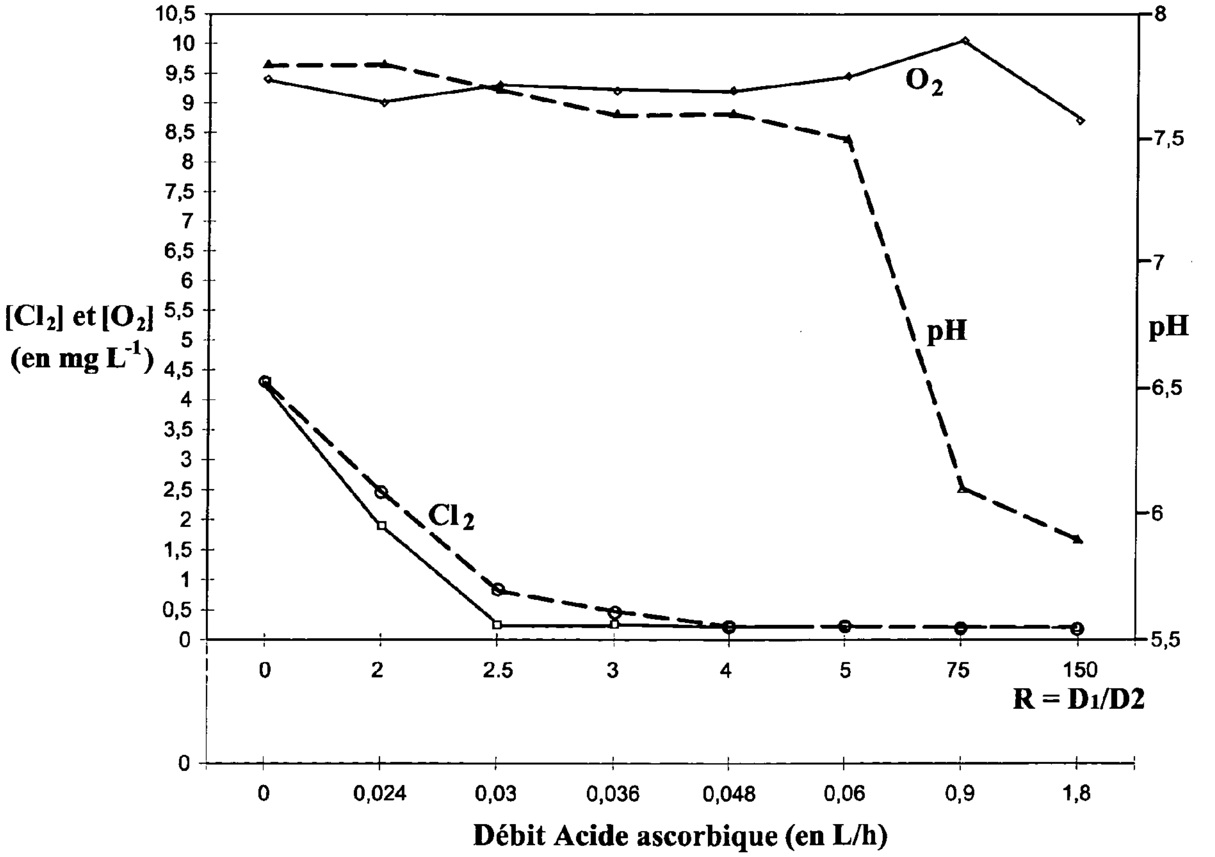

[Cl2] et [O2] (en mg L-1)
pH
O2
pH
Cl2
10,5
10
9,5
9
8,5
8
7,5
7
6,5
6
5,5
5
4,5
4
3,5
3
2,5
2
1,5
1
0,5
0
8
7,5
7
6,5
6
5,5
0
2
2.5
3
4
5
75
150
R = D1/D2
0
0
0,024
0,03
0,036
0,048
0,06
0,9
1,8
Débit Acide ascorbique (en L/h)

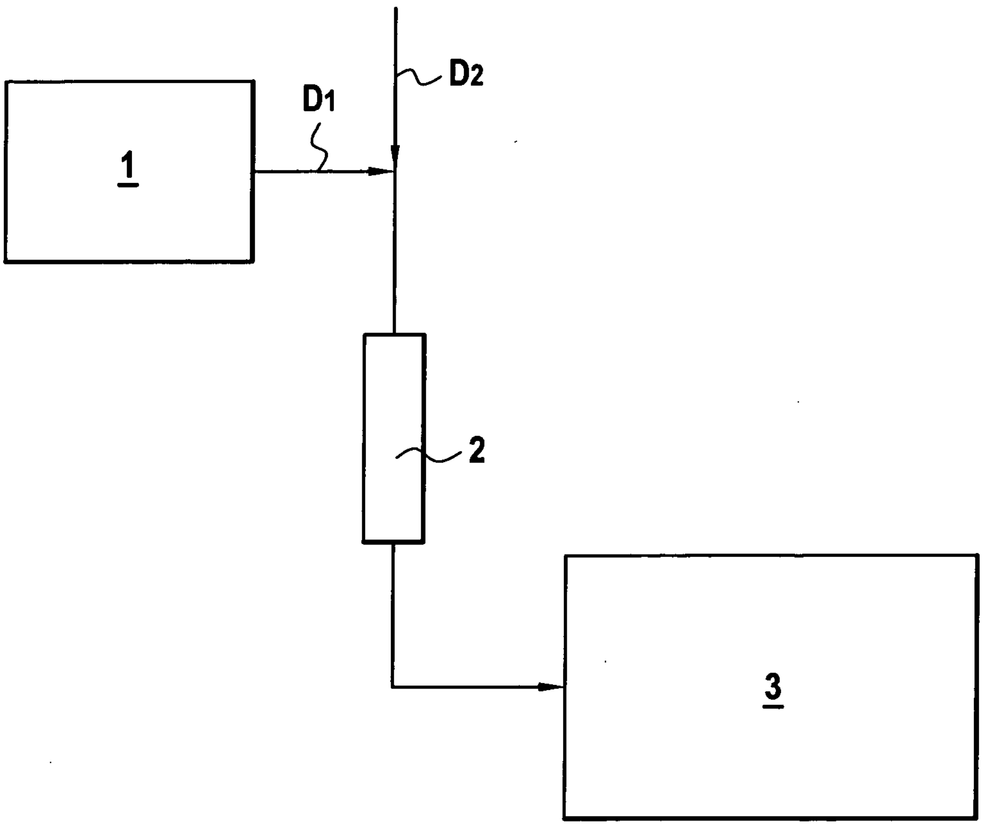


FIG.3

Office européen des brevets

# RAPPORT DE RECHERCHE EUROPEENNE

Numéro de la demande
EP 06 35 8010

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | US 2004/069719 A1 (WANG DESHENG) 15 avril 2004 (2004-04-15) * alinéas [0009], [0013], [0015], [0019], [0021] * | 1,2,4-13 | INV. G01N33/18 |
| Y | US 2002/125198 A1 (SIMPSON GREGORY D) 12 septembre 2002 (2002-09-12) * alinéas [0001] - [0005], [0049] - [0053], [0056], [0058]; revendications 1,4,5,8,10,11,13 * | 1,2,4,6, 8-10 | |
| Y | PATENT ABSTRACTS OF JAPAN vol. 1999, no. 12, 29 octobre 1999 (1999-10-29) -& JP 11 178733 A (SEKISUI CHEM CO LTD), 6 juillet 1999 (1999-07-06) * abrégé * | 1,4,5,7, 11,13 | |
| Y | PATENT ABSTRACTS OF JAPAN vol. 2002, no. 03, 3 avril 2002 (2002-04-03) -& JP 2001 327852 A (MATSUSHITA ELECTRIC IND CO LTD), 27 novembre 2001 (2001-11-27) * abrégé * | 1,4,5,7 | DOMAINES TECHNIQUES RECHERCHES (IPC) |
| Y | US 6 328 896 B1 (ATNOOR DEVENDRA ET AL) 11 décembre 2001 (2001-12-11) * colonne 9, ligne 53-56; figures 2a,3; exemples * | 5,7 | C02F G01N |
| Y | US 4 500 510 A (GOLDSTEIN ET AL) 19 février 1985 (1985-02-19) * colonne 4, ligne 9-16 * * colonne 6, ligne 8-22; revendication 8; exemple 4 * | 11-13 | |
| A | US 2004/147030 A1 (NEBERT DANIEL W [US]) 29 juillet 2004 (2004-07-29) * le document en entier * | 1-13 | |

Le présent rapport a été établi pour toutes les revendications

3

| Lieu de la recherche | Date d'achèvement de la recherche | Examinateur |
|---|---|---|
| Munich | 1 septembre 2006 | Kurtulan, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 06 35 8010

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

01-09-2006

| Document brevet cité au rapport de recherche | Date de publication | Membre(s) de la famille de brevet(s) | Date de publication |
|---|---|---|---|
| US 2004069719 A1 | 15-04-2004 | AUCUN | |
| US 2002125198 A1 | 12-09-2002 | AUCUN | |
| JP 11178733 A | 06-07-1999 | AUCUN | |
| JP 2001327852 A | 27-11-2001 | AUCUN | |
| US 6328896 B1 | 11-12-2001 | EP 1073611 A1 | 07-02-2001 |
| | | JP 2002512883 T | 08-05-2002 |
| | | WO 9955622 A1 | 04-11-1999 |
| US 4500510 A | 19-02-1985 | AUCUN | |
| US 2004147030 A1 | 29-07-2004 | US 2006143718 A1 | 29-06-2006 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2573875